# EUROPÄISCHE PATENTSCHRIFT

(11) **EP 4 245 294 B1**
(45) Veröffentlichungstag und Bekanntmachung des Hinweises auf die Patenterteilung: **12.03.2025**
(21) Anmeldenummer: 22162502.3
(22) Anmeldetag: 16.03.2022
(51) Int. Cl.: A61K 9/00, A61K 9/06, A61K 31/00

(54) **KATHETERGLEITGEL MIT ERHÖHTER STABILITÄT**
CATHETER SLIDE WITH ENHANCED STABILITY
GEL LUBRIFIANT POUR CATHÉTER À STABILITÉ ÉLEVÉE

(43) Veröffentlichungstag der Anmeldung: 20.09.2023
(73) Patentinhaber: Pharmazeutische Fabrik Montavit Ges.m.b.H., 6067 Absam/Tirol (AT)
(72) Erfinder: Knoflach, Daniel, 6067 Absam/Tirol (AT)
(74) Vertreter: Frick, Robert

(56) Entgegenhaltungen:
- WO-A1-2015/074730
- WO-A1-2020/136502
- WO-A2-2006/099359

## Beschreibung

Die Erfindung betrifft eine Gelzusammensetzung zur topischen medizinischen Verwendung als Gleitmittel, insbesondere als Kathetergleitgel. Die Gelzusammensetzung enthält ein Antiseptikum und vorzugsweise ferner ein Anästhetikum.

Im Rahmen mancher medizinischen Behandlungen ist es erforderlich, medizinische Geräte wie Katheter, Endoskope, Sonden oder andere medizinische Geräte in Körperöffnungen wie beispielsweise die Harnröhre eines Patienten einzuführen. Im Allgemeinen sind diese Maßnahmen für den Patienten unangenehm oder gar schmerzhaft, und es sind auch Verletzungen an den jeweiligen Körperöffnungen möglich. Zur Minderung dieser Unannehmlichkeiten, zur Verhinderung von Verletzungen und generell zur Vereinfachung dieser Maßnahmen ist es üblich, ein Gleitmittel vorab in die entsprechende Körperöffnung zu instillieren oder gegebenenfalls auch auf die Geräte aufzutragen, bevor diese in die Körperöffnung eingeführt werden. Unter anderem kommen hierbei Hydrogele zum Einsatz, die wirkstofffrei sein können oder mit Lokalanästhetika wie beispielsweise Lidocain und Antiseptika wie beispielsweise Chlorhexidin versetzt sein können.

Bei derartigen Gleitmitteln kann es aber zu Problemen mit der Stabilität kommen. Beispielsweise können sich Wirkstoffe mit der Zeit zersetzen. So kann beispielsweise das aus unterschiedlichen Gründen für den betreffenden Einsatz geeignete Chlorhexidin unter entsprechenden Bedingungen als Abbauprodukt das bedenkliche 4-Choranilin bilden. Weiterhin können Antiseptika mit Wirkstoffen wie Lokalanästhetika interagieren bzw. zumindest deren Löslichkeit im Gel senken. So interagiert beispielsweise Chlorhexidin mit manchen Salzen wie beispielsweise Lidocain-Hydrochlorid in einer Weise, die das Löslichkeitsprodukt dieser Stoffe erheblich reduziert und zu unerwünschten Fällungsreaktionen führt. Reaktive Antiseptika wie Chlorhexidin können zwar durch andere Antiseptika, beispielsweise durch Parabene ersetzt werden, dies geht jedoch in der Regel auf Kosten der Effektivität und auch Verträglichkeit bzw. Unbedenklichkeit.

Im Stand der Technik sind aus der WO 2006/099259 A2 Kathetergleitgele bekannt, die Lidocain, Chlorhexidin und Butylenglykol enthalten. Auch die WO 2015/074730 A1 und die WO 2020/136502 A1 beschreiben Kathetergleitgele umfassend gelöste Lokalanesthätika und Antiseptika.

Aufgabe der Erfindung ist es, eine gattungsgemäße galenische Gelzusammensetzung bereitzustellen, die ein Antiseptikum und vorzugsweise ferner ein Anästhetikum aufweist und in dem die genannten unerwünschten Effekte reduziert sind und dessen Stabilität und Haltbarkeit folglich verbessert sind. Ziel ist die signifikante Steigerung der Leistungsfähigkeit und Sicherheit entsprechender Produkte gegenüber dem Stand der Technik.

Vor diesem Hintergrund betrifft die Erfindung eine Gelzusammensetzung, die ein Antiseptikum und vorzugsweise ferner ein Anästhetikum aufweist, wobei die Gelzusammensetzung ferner eine Diol-Komponente enthält, bei der es sich um Hexylenglykol, Pentylenglycol oder Strukturisomere davon handelt.

Vermutlich auf Grund seiner chemischen Struktur und seiner oberflächenaktiven Eigenschaft führt die wie oben definierte Diol-Komponente zu einer besseren Löslichkeit des Anästhetikums und des Antiseptikums und zu einer Erhöhung der Stabilität der Wirkstoffe sowie auch der physikochemischen Eigenschaften der galenischen Gelzusammensetzung. Dieser Effekt konnte mit anderen, insbesondere niedrigkettigen Polyolen wie Glycerol oder Propylenglycol nicht erreicht werden.

Bei der Gelzusammensetzung handelt es sich vorzugsweise um ein Hydrogel. Als gelbildende hydrophile Polymere können beispielsweise Polyalkohole natürlichen oder synthetischen Ursprungs zum Einsatz kommen. Bevorzugte Beispiele umfassen Cellulosederivate wie Hydroxyethylcellulose.

Der Wassergehalt (exklusive der darin gelösten Inhaltsstoffe) macht typischerweise 40-99% der Gesamtmasse des Hydrogels aus. Der Gehalt an gelbildenden Polymeren gemessen an der Gesamtmasse der feuchten Gelzusammensetzung liegt vorzugsweise zwischen 0,1 Gew.-% und 5 Gew.-%, weiter vorzugsweise zwischen 0,5 Gew.-% und 3 Gew.-%.

Bei dem Anästhetikum handelt es sich vorzugsweise um ein topisches Lokalanästhetikum vom Ester-Typ oder Aminoamid-Typ. Beispiele hierfür sind Benzocain, Procain Tetracain (alle Ester-Typ) oder Lidocain, Prilocain, Etidocain, Ropivacain, Mepivacain, Bupivacain (alle Amid-Typ). Ein besonders bevorzugtes Beispiel umfasst Lidocain.

Der Gehalt an Anästhetikum gemessen an der Gesamtmasse der feuchten Gelzusammensetzung liegt vorzugsweise zwischen 0,1 Gew.-% und 7 Gew.-%, weiter vorzugsweise zwischen 0,5 Gew.-% und 5 Gew.-%.

Bei dem Antiseptikum handelt es sich vorzugsweise um ein Guanidin-Derivat, insbesondere eine Biguanid-Verbindung. Beispiele umfassen Chlorhexidin, Polyhexanid oder Hexamidin. Besonders bevorzugt ist der Einsatz von Chlorhexidin.

Der Gehalt an Antiseptikum gemessen an der Gesamtmasse der feuchten Gelzusammensetzung liegt vorzugsweise zwischen 0,01 und 4 Gew.-%, vorzugsweise zwischen 0,02 und 2 Gew.-%, weiter vorzugsweise zwischen 0,03 und 1 Gew.-%.

Gemäß der Erfindung handelt es sich bei der Diol-Komponente um Hexylenglykol, Pentylenglycol und strukturisomere Alkandiole dieser Verbindungen. Besonders hervorzuheben ist Hexylenglykol.

Der Gehalt an der Diol-Komponente gemessen an der Gesamtmasse der feuchten Gelzusammensetzung liegt vorzugsweise bei zwischen 5 Gew.-% und 50 Gew.-%, weiter vorzugsweise bei zwischen 10 Gew.-% und 30 Gew.-%.

Vorzugsweise liegen das Antiseptikum und, sofern vorhanden, das Anästhetikum in der Gelzusammensetzung hauptsächlich als Salze einer Hydroxycarbonsäure in der Gelzusammensetzung vor.

Bei der Hydroxycarbonsäure kann es sich um eine α-Hydroxycarbonsäure handeln, wobei Kohlenstoffzahlen von kleiner sechs bevorzugt sind. Beispiele umfassen Glykolsäure oder Milchsäure. Besonders hervorzuheben ist Milchsäure. Die Säure kann teilweise als Salz und insbesondere als Mischung aus Salz und freier Säure und mithin Puffer(bestandteil) in der Gelzusammensetzung vorliegen.

Gängige Praxis war bisher in gattungsgemäßen Gelzusammensetzungen die pH-Einstellung mit Salzsäure. Der Eintrag der zusätzlichen Chloridionen, bzw. im allgemeinen anorganischer Ionen wie etwa Phosphationen, hat jedoch potentiell die Bildung schwerlöslicher Salze mit dem Antiseptikum zur Folge. Vorliegend kann vorgesehen sein, dass die Wirkstoffe als freie Base zugegeben werden und in der Zusammensetzung mit der Hydroxycarbonsäure die Salze bilden. Der Gehalt an anorganischen Ionen soll möglichst weit reduziert werden. Eine geringe Anzahl kann jedoch toleriert werden, solange die Hydroxycarbonsäure in ausreichender Konzentration vorliegt.

Vorzugsweise weist die Gelzusammensetzung weniger als 1,5 Gew.-%, weiter vorzugsweise weniger als 1,0 Gew.-% Chlorid bzw. generell anorganische Anionen auf.

Der Gehalt an Hydroxycarbonsäure gemessen an der Gesamtmasse der feuchten Gelzusammensetzung liegt vorzugsweise bei zwischen 0,01 Gew.-% und 10 Gew.-%, weiter vorzugsweise bei zwischen 0,5 Gew.-% und 5 Gew.-%.

Der pH-Wert der Gelzusammensetzung wird vorzugsweise durch einen Puffer eingestellt, der teilweise oder vollständig aus der Hydroxycarbonsäure und einem Salz der Hydroxycarbonsäure (wobei es sich bei dem Kation zumindest teilweise um das Antiseptikum oder, sofern vorhanden, Anästhetikum handeln kann) gebildet ist. Beispiele umfassen Lactatpuffer, Glycolatpuffer oder Kombinationen daraus.

Der pH-Wert der Gelzusammensetzung liegt vorzugsweise in einem Bereich von pH 3 bis pH 8, vorzugsweise bei pH 4 bis pH 7. Davon abweichende pH-Werte begünstigen Fällungsreaktionen der enthaltenen Bestandteile, insbesondere Antiseptika oder Anästhetika größer. Zudem liegt der natürliche pH im Urogenitaltrakt bei etwa pH 4 bis pH 7, wobei in den genannten Bereichen für entsprechende Anwendungen eine optimale Verträglichkeit gewährleistet ist.

In der erfindungsgemäßen Gelzusammensetzung liegen die Wirkstoffe, insbesondere das Antiseptikum und das gegebenenfalls vorhandene Anästhetikum vorzugsweise in Lösung vor. Mit anderen Worten ist bevorzugt, dass keine Emulsionspartikel oder feste Trägerpartikel in der Gelzusammensetzung vorliegen, welche die Wirkstoffe tragen bzw. beinhalten. Die Erfindung betrifft also vorzugsweise ein einphasiges System. Die Möglichkeit der Bereitstellung einer stabilen Zusammensetzung als Lösung wird durch die Diol-Komponente begünstigt.

Die Erfindung betrifft weiterhin die Verwendung einer erfindungsgemäßen Gelzusammensetzung zum Instillieren in Körperöffnungen, insbesondere in die Harnröhre, oder zur Auftragung auf Bereiche medizinischer Geräte, deren bestimmungsgemäße Verwendung die Einführung in eine Körperöffnung, insbesondere die Harnröhre, das Rektum oder den Bronchialraum, oder die Verwendung beim Setzen eines suprapubischen Katheters umfasst. Besonders bevorzugt ist die Verwendung als Kathetergleitgel.

Weitere Einzelheiten und Vorteile der Erfindung ergeben sich aus dem nachfolgend beschriebenen Ausführungsbeispiel.

Ein Kathetergleitgel wird mit den in Tabelle 1 angeführten Inhaltsstoffen hergestellt.

**Tabelle 1: Zusammensetzung**

| **Rohstoff** | **g/100g** |
|---|---|
| Wasser [g] | 70,22 |
| Chlorhexidin*2HCl [g] | 0,05 |
| Na-Lactat-Lsg 50% [g] | 6,00 |
| Lidocain [g] freie Base | 1,73 |
| HEC [g] | 2,00 |
| Hexyleneglykol [g] | 20,00 |
| LacAc 90% [g] | q.s. |
| NaOH [ml] | q.s. |
| Gesamtmenge Lösung | 100 |

Ein ähnliches Kathetergleitgel mit anderem Puffer, mit Lidocain als Hydrochlorid und insbesondere ohne Hexylenglykol gehört zum Stand der Technik und wird von der Pharmazeutischen Fabrik Montavit Ges.m.b.H. unter dem Handelsnamen Cathejell Lidocain C vertrieben.

In der wie oben beschriebenen Zusammensetzung wurde überraschenderweise beobachtet, dass das Hexylenglykol einen positiven Einfluss auf die Haltbarkeit und die Stabilität der Gelzusammensetzung hat. Insbesondere kann die Löslichkeit von Chlorhexidin deutlich erhöht werden. Eine mögliche Erklärung besteht darin, dass die höheren Glykole, beginnend insbesondere bei einer Kohlenstoffzahl von vier, aufgrund ihrer unpolaren Kohlenstoffkette sowie der polaren Hydroxylgruppen hierfür als Lösungsvermittler geeignet sind. Außerdem haben diese Substanzen eine gute Gewebeverträglichkeit und in entsprechender Konzentration hautpflegende Eigenschaften. Durch die Verwendung von Lidocain als freier Base wird zudem ein Eintrag von Chloridionen vermieden, was die Entstehung von schwer löslichen Chlorhexidinkristallen verhindert.

## Patentansprüche

1. Gelzusammensetzung, die ein Antiseptikum und vorzugsweise ferner ein Anästhetikum aufweist,
**dadurch gekennzeichnet,**
**dass** die Gelzusammensetzung ferner eine Diol-Komponente enthält, bei der es sich um Hexylenglykol, Pentylenglycol oder Strukturisomere davon handelt.

2. Gelzusammensetzung nach Anspruch 1, **dadurch gekennzeichnet, dass** es sich bei der Gelzusammensetzung um ein Hydrogel handelt, dessen Wassergehalt vorzugsweise bei zwischen 40 und 99 Gew.-% liegt.

3. Gelzusammensetzung nach einem der vorhergehenden Ansprüche, **dadurch gekennzeichnet, dass** es sich bei dem Anästhetikum um ein topisches Lokalanästhetikum vom Ester-Typ oder dem Aminoamid-Typ, insbesondere dem Aminoamid-Typ, und besonders bevorzugt um Lidocain handelt.

4. Gelzusammensetzung nach einem der vorhergehenden Ansprüche, **dadurch gekennzeichnet, dass** der Gehalt an Anästhetikum gemessen an der Gesamtmasse der feuchten Gelzusammensetzung bei zwischen 0,1 Gew.-% und 7 Gew.-%, vorzugsweise bei zwischen 0,5 Gew.-% und 5 Gew.-% liegt.

5. Gelzusammensetzung nach einem der vorhergehenden Ansprüche, **dadurch gekennzeichnet, dass** es sich bei dem Antiseptikum um ein Guanidin-Derivat, vorzugsweise um Chlorhexidin, Polyhexanid oder Hexamidin, und besonders bevorzugt um Chlorhexidin handelt.

6. Gelzusammensetzung nach einem der vorhergehenden Ansprüche, **dadurch gekennzeichnet, dass** der Gehalt an Antiseptikum gemessen an der Gesamtmasse der feuchten Gelzusammensetzung bei zwischen 0,01 und 4 Gew.-%, vorzugsweise bei zwischen 0,02 und 2 Gew.-%, weiter vorzugsweise bei zwischen 0,03 und 1 Gew.-% liegt.

7. Gelzusammensetzung nach einem der vorhergehenden Ansprüche, **dadurch gekennzeichnet, dass** der Gehalt an der Diol-Komponente gemessen an der Gesamtmasse der feuchten Gelzusammensetzung bei zwischen 5 Gew.-% und 50 Gew.-%, vorzugsweise bei zwischen 10 Gew.-% und 30 Gew.-% liegt.

8. Gelzusammensetzung nach einem der vorhergehenden Ansprüche, **dadurch gekennzeichnet, dass** das Antiseptikum und, sofern vorhanden, das Anästhetikum hauptsächlich als Salze einer Hydroxycarbonsäure in der Gelzusammensetzung vorliegen.

9. Gelzusammensetzung nach Anspruch 8, **dadurch gekennzeichnet, dass** es sich bei der Hydroxycarbonsäure um eine α-Hydroxycarbonsäure handelt, vorzugsweise eine α-Hydroxycarbonsäure mit weniger als sechs Kohlenstoffatomen, und besonders bevorzugt um Glykolsäure oder Milchsäure.

10. Gelzusammensetzung nach einem der vorhergehenden Ansprüche, **dadurch gekennzeichnet, dass** die Gelzusammensetzung weniger als 1,5 Gew.-%, vorzugsweise weniger als 1,0 Gew.-% Chlorid und vorzugsweise generell anorganische Anionen aufweist.

11. Gelzusammensetzung nach einem der vorhergehenden Ansprüche, **dadurch gekennzeichnet, dass** der pH-Wert der Gelzusammensetzung bei **pH** 3 bis **pH 8,** vorzugsweise bei pH 4 bis pH 7 liegt.

12. Gelzusammensetzung nach einem der vorhergehenden Ansprüche, **dadurch gekennzeichnet, dass** das Antiseptikum und das gegebenenfalls vorhandene Anästhetikum gelöst in der Gelzusammensetzung vorliegen.

13. Gelzusammensetzung nach einem der vorhergehenden Ansprüche zur Verwendung zum Instillieren in Körperöffnungen, insbesondere in die Harnröhre, oder zur Auftragung auf Bereiche medizinischer Geräte, deren bestimmungsgemäße Verwendung die Einführung in eine Körperöffnung, insbesondere die Harnröhre, das Rektum oder den Bronchialraum, oder die Verwendung beim Setzen eines suprapubischen Katheters umfasst.

14. Gelzusammensetzung nach einem der Ansprüche 1-12 zur Verwendung nach Anspruch 13, **dadurch gekennzeichnet, dass** die Gelzusammensetzung als Kathetergleitgel eingesetzt wird.

## Claims

1. Gel composition comprising an antiseptic agent and preferably also an anaesthetic agent,
**characterised in that**
the gel composition further contains a diol component, which is hexylene glycol, pentylene glycol or structural isomers.

2. Gel composition according to claim 1, **characterised in that** the gel composition is a hydrogel, the water content of which is preferably between 40 and 99 wt.%.

3. Gel composition according to any of the preceding claims, **characterised in that** the anaesthetic agent is a topical local anaesthetic of the ester type or the amino-amide type, in particular the amino-amide type, and is particularly preferably lidocaine.

4. Gel composition according to any of the preceding claims, **characterised in that** the content of anaesthetic agent, measured on the basis of the total mass of the moist gel composition, is between 0.1 wt.% and 7 wt.%, preferably between 0.5 wt.% and 5 wt.%.

5. Gel composition according to any of the preceding claims, **characterised in that** the antiseptic agent is a guanidine derivative, preferably chlorhexidine, polyhexanide or hexamidine, and particularly preferably chlorhexidine.

6. Gel composition according to any of the preceding claims, **characterised in that** the content of anaesthetic agent, measured on the basis of the total mass of the moist gel composition, is between 0.01 and 4 wt.%, preferably between 0.02 and 2 wt. %, more preferably between 0.03 and 1 wt.%.

7. Gel composition according to any of the preceding claims, **characterised in that** the content of the diol component, measured on the basis of the total mass of the moist gel composition, is between 5 wt.% and 50 wt.%, preferably between 10 wt.% and 30 wt.%.

8. Gel composition according to any of the preceding claims, **characterised in that** the antiseptic agent and, if provided, the anaesthetic agent are mainly present as salts of a hydroxycarboxylic acid in the gel composition.

9. Gel composition according to claim 8, **characterised in that** the hydroxycarboxylic acid is an ?-hydroxycarboxylic acid, preferably an ?-hydroxycarboxylic acid having less than six carbon atoms, and particularly preferably is glycolic acid or lactic acid.

10. Gel composition according to any of the preceding claims, **characterised in that** the gel composition comprises less than 1.5 wt.%, preferably less than 1.0 wt.%, chloride and preferably generally inorganic anions.

11. Gel composition according to any of the preceding claims, **characterised in that** the pH of the gel composition is pH 3 to pH 8, preferably pH 4 to pH 7.

12. Gel composition according to any of the preceding claims, **characterised in that** the antiseptic agent and the potentially provided anaesthetic agent are present in a form dissolved in the gel composition.

13. Gel composition according to any of the preceding claims for use for instilling in body orifices, in particular in the urethra, or for application to regions of medical devices, the intended use of which involves insertion into a body orifice, in particular the urethra, the rectum or the bronchial system, or for use when inserting a suprapubic catheter.

14. Gel composition according to any of claims 1-12 for use according to claim 13, **characterised in that** the gel composition is used as a catheter lubricant gel.

## Revendications

1. Composition de gel, qui présente un antiseptique et de préférence en outre un anesthésique,
**caractérisée en ce que**
la composition de gel contient en outre un composant diol qui consiste en l'hexylène glycol, le pentylène glycol ou des isomères structurels de ceux-ci.

2. Composition de gel selon la revendication 1, **caractérisée en ce que** la composition de gel consiste en un hydrogel dont la teneur en eau est de préférence comprise entre 40 et 99 % en poids.

3. Composition de gel selon l'une quelconque des revendications précédentes, **caractérisée en ce que** l'anesthésique consiste en un anesthésique local topique de type ester ou de type aminoamide, notamment de type aminoamide, et de manière particulièrement préférée en la lidocaïne.

4. Composition de gel selon l'une quelconque des revendications précédentes, **caractérisée en ce que** la teneur en anesthésique, mesurée par rapport à la masse totale de la composition de gel humide, est comprise entre 0,1 % en poids et 7 % en poids, de préférence entre 0,5 % en poids et 5 % en poids.

5. Composition de gel selon l'une quelconque des revendications précédentes, **caractérisée en ce que** l'antiseptique consiste en un dérivé de guanidine, de préférence en la chlorhexidine, le polyhexanide ou l'hexamidine, et de manière particulièrement préférée en la chlorhexidine.

6. Composition de gel selon l'une quelconque des revendications précédentes, **caractérisée en ce que** la teneur en antiseptique, mesurée par rapport à la masse totale de la composition de gel humide, est comprise entre 0,01 et 4 % en poids, de préférence entre 0,02 et 2 % en poids, plus préférentiellement entre 0,03 et 1 % en poids.

7. Composition de gel selon l'une quelconque des revendications précédentes, **caractérisée en ce que** la teneur en composant diol, mesurée par rapport à la masse totale de la composition de gel humide, est comprise entre 5 % en poids et 50 % en poids, de préférence entre 10 % en poids et 30 % en poids.

8. Composition de gel selon l'une quelconque des revendications précédentes, **caractérisée en ce que** l'antiseptique et, s'il est présent, l'anesthésique sont principalement présents sous forme de sels d'un acide hydroxycarboxylique dans la composition de gel.

9. Composition de gel selon la revendication 8, **caractérisée en ce que** l'acide hydroxycarboxylique consiste en un acide α-hydroxycarboxylique, de préférence un acide α-hydroxycarboxylique ayant moins de six atomes de carbone, et de manière particulièrement préférée en l'acide glycolique ou l'acide lactique.

10. Composition de gel selon l'une quelconque des revendications précédentes, **caractérisée en ce que** la composition de gel présente moins de 1,5 % en poids, de préférence moins de 1,0 % en poids de chlorure et, de préférence, généralement des anions inorganiques.

11. Composition de gel selon l'une quelconque des revendications précédentes, **caractérisée en ce que** le pH de la composition de gel se situe de pH 3 à pH 8, de préférence de pH 4 à pH 7.

12. Composition de gel selon l'une quelconque des revendications précédentes, **caractérisée en ce que** l'antiseptique et l'anesthésique éventuellement présent se présentent sous forme dissoute dans la composition de gel.

13. Composition de gel selon l'une quelconque des revendications précédentes pour utilisation pour l'instillation dans des orifices corporels, notamment dans l'urètre, ou pour l'application sur des zones de dispositifs médicaux dont l'utilisation prévue comprend l'introduction dans un orifice corporel, notamment l'urètre, le rectum ou l'espace bronchique, ou l'utilisation lors de la pose d'un cathéter sus-pubien.

14. Composition de gel selon l'une quelconque des revendications 1 à 12 pour utilisation selon la revendication 13, **caractérisée en ce que** la composition de gel est utilisée en tant que gel lubrifiant pour cathéter.
